Europäisches Patentamt

European Patent Office

Office européen des brevets

(19)

(11) **EP 1 158 087 A1**

## (12) EUROPEAN PATENT APPLICATION

(43) Date of publication:
**28.11.2001 Bulletin 2001/48**

(51) Int Cl.[7]: **D06M 10/02**, D06M 15/643, D06M 10/10

(21) Application number: **00850022.5**

(22) Date of filing: **04.02.2000**

(84) Designated Contracting States:
**AT BE CH CY DE DK ES FI FR GB GR IE IT LI LU MC NL PT SE**
Designated Extension States:
**AL LT LV MK RO SI**

(71) Applicants:
- **SCA Hygiene Products AB**
  **405 03 Göteborg (SE)**
- **L'AIR LIQUIDE, SOCIETE ANONYME POUR L'ETUDE ET L'EXPLOITATION DES PROCEDES GEORGES CLAUDE**
  **75321 Paris Cédex 07 (FR)**
- **SOFTAL electronic GmbH**
  **21107 Hamburg (DE)**

(72) Inventors:
- **Nihlstrand, Anna**
  **431 35 Mölndal (SE)**
- **Abbas, Shabira**
  **422 42 Göteborg (SE)**

- **Moberg-Alehammar, Barbro**
  **431 39 Mölndal (SE)**
- **Johansson, Asa**
  **417 16 Göteborg (SE)**
- **Martens, Bernd,2 c/o Softal Electronic**
  **21107 Hamburg (DE)**
- **Prinz, Eckhard, c/o Softal Electronic**
  **21107 Hamburg (DE)**
- **Forster, Franck, c/o Softal Electronic**
  **21107 Hamburg (DE)**
- **Villermet, Alain**
  **78220 Viroflay (FR)**
- **Cocollos, Panayotis**
  **78830 Bullion (FR)**
- **Coeuret, Francois**
  **78280 Guyancourt (FR)**

(74) Representative: **Romare, Laila Anette**
**Albihns Göteborg AB**
**Box 142**
**401 22 Göteborg (SE)**

(54) **Fibrous structure**

(57)    The present invention relates to a fibrous structure, being testable in a series of run-off tests, wherein each run-off test comprises exposing said fibrous structure to a volume of a test solution and said fibrous structure initially is hydrophobic and has been treated to be hydrophilic. The fibrous structure exhibits a run-off level that is less than five percent by weight throughout a series of run-off tests wherein the series starts with a first run-off test and ends with a fifth run-off test. The volume of the test solution is 25 ml and the test solution after the first run-off test has a surface tension which is less than $60*10^{-3}$ N/m. The fibrous structure can be used for non-woven and tissue paper product.

*FIG. 1*

EP 1 158 087 A1

**Description**

TECHNICAL FIELD

The present invention provides a fibrous structure which exhibits excellent properties of "hydrophilicity" or "wettability", wherein the properties of "hydrophilicity" or "wettability" are both immediate and durable.

Further, the present invention relates to a fibrous structure (either of a woven or a non-woven type, and of a natural or synthetic material), being testable in a series of run-off tests, wherein each run-off test comprises exposing said fibrous structure to a volume of a test solution and said fibrous structure initially is hydrophobic and has been treated to be hydrophilic.

Moreover, the present invention relates to a fibrous structure having one or more types of polar, silicon-containing compounds bounded to at least one portion of the surface of said fibrous structure.

In accordance with the fibrous structure of the invention, properties of hydrophilicity will be particularly looked for.

Taking the example of an absorbent article comprising fibrous structures made of non-woven materials, the fibrous structures are produced from comparatively hydrophobic synthetic fibres such as, for example, fibres of polypropylene or polyethylene which are treated in order to make the materials liquid permeable.

BACKGROUND ART

In order to obtain fluid absorbent articles which exhibit good wicking ability, a high total and local fluid uptake capacity, good fluid retaining capacity and a high degree of surface dryness, such articles are usually built up of a plurality of different fibrous structures having different functions. The fibrous structures are often produced of inherently hydrophobic materials, e.g. nonwoven materials, and thus such inherently hydrophobic fibrous structures need to be modified to allow fluid uptake. One major problem when constructing fluid absorbent articles of this kind is, however, that it is difficult to obtain optimal wettability, i.e. an optimal degree of hydrophilicity, which is both immediate and durable, i.e. remains unchanged after the article has been exposed to wetting. Furthermore, it is difficult to maintain stable wetting characteristics in absorbent articles, which are stored for an extended period of time.

Regarding fluid permeable cover sheets, which are suitable made of said fibrous structures, for use in absorbent articles such as diapers, incontinence guards and sanitary napkins, wherein the cover sheet is intended to be in contact with the body of a user during use, it is important that the cover sheet immediately exhibits a desired wettability and could stand repeated wettings. In other words, the cover sheet should immediately exhibit an optimal degree of hydrophilicity and remain fluid permeable even after the absorbent article has been exposed to fluid impact several times. Furthermore, it is important that the cover sheet can accept a large amount of fluid during a short interval of time. Another important property of the fluid permeable cover sheet is the ability to exhibit high surface dryness even after having been exposed to several wettings. In order to obtain a cover sheet having the desired properties, it is important that the cover sheet immediately exhibits an optimal, i.e. a desired, degree of hydrophilicity and that the degree of hydrophilicity varies only within a very limited range when the fibrous structure is wetted or when it is subjected to ageing.

As well known to the man skilled in the art, the literature of these fields talk about the properties of "hydrophilicity" or "wettability" of a substrate and often report measurements of "run-off' and "surface tension" to evaluate such properties.

Commonly used methods for increasing the wettability of fluid permeable fibrous structure for use as cover sheets in absorbent articles (the same applies to any fibrous structure to be used in absorbent articles) are:

Treatment of the fibrous structure with surface active agents, wherein an increased amount of surface active agents has been used to further increase said wettability. Further, treatment with surface active agents here includes use of mixtures of surfactants, e.g. a mixture of a co-wetting agent (reducing surface tension of the fluid which leads to a rapid inlet) and a surfactant having a more durable character, or addition of surface active agents as internal additives.

Exposure of the material of said fibrous structure to a corona treatment in combination with any addition of hydrophilic substances, methods for corona treatment are for example described in US 5,576,076, US 5,527,629 and US 5,523,124.

The treatment of the fibrous structures with surface active agents is for example carried out by coating the hydrophobic material with a surface active agent. In order for a material to be fluid permeable the contact angle between the surface of the material and the fluid must be less than 90°. However, a problem in connection with using fibrous structure which have been coated with a surface active agent is that such fibrous structure exhibit decreasing fluid permeability with repeated wetting. The reason for this is that the applied surface active agents are not firmly attached to the surface of the cover material and will be detached from the cover material and solved in body fluid during the first wetting. At subsequent wetting the amount of surface active agent which remains on the surface of the cover sheet is considerably reduced, resulting in impaired fluid permeability. The same applies to absorbent articles when said surface active agents are added as internal additives, because surface active agents added as internal additives do not migrate fast enough to the surface of the absorbent article after wettings of said surface.

Further problems with the treatment of the fibrous structure with surface active agents, besides the lacking durability, are problems which also increases with increased amount of added surface active agent, for example skin irritation which is caused by migration of surface active agents from the cover sheet to the skin of the user, and influence of the absorbent core comprised in said absorbent articles also because of said migration of surface active agents.

Further, commonly used air corona treatment (optionally in combination with addition of hydrophilic substances) does not render any absorbent articles exhibiting an optimal, i.e. a desired, degree of hydrophilicity which is durable. Said degree of hydrophilicity is not durable since said commonly used corona treatment may create a low molecular weight material which is not properly bound to the surface. Hence subsequent wetting of the surface of the cover sheet may partly remove the created low molecular weight material and added hydrophilic substances and thus resulting in impaired fluid permeability.

In US, A, 5814567 a durable hydrophilic coating for a porous hydrophobic substrate is desribed, which may include exposing the substrate to a field of reactive species.

Moreover at a first wetting by a fluid, in US, A, 5814567 said hydrophilic coating does not decrease the surface tension of said fluid.

European patent application 98402010.7 (not published) describes fibrous structures which exhibits a well defined rate of wetting and may be useful in absorbent articles. The fibrous structures of the European patent application 98402010.7 have one or more types of polar, silicon-containing compounds bounded to their surfaces but said fibrous structures are not treated with any surface active agent, i.e. no surface active agent at all is added. Thus absorbent articles comprising the fibrous structures described in European patent application 98402010.7 do not show any of the above described problems related to conventional surface active agent treatment or to commonly used corona treatment (optionally in combination with addition of hydrophilic substances).

Nevertheless there is still a need for further modifications of fibrous structures comprised in absorbent articles not showing any of or at least significantly reducing the problems related to conventional surface active agent treatment or to commonly used corona treatment (optionally in combination with addition of hydrophilic substances), especially there is a need for such absorbent articles exhibiting excellent properties of "hydrophilicity" or "wettability" which are both immediate and durable.

DISCLOSURE OF THE INVENTION

The present invention provides a fibrous structures which exhibiting excellent properties of "hydrophilicity" or "wettability", wherein the properties of "hydrophilicity" or "wettability" are both immediate and durable. A fibrous structure, or any material structure, acts hydrophobic when a contact angle between said structure and water is greater than 90 ° and/or when a contact angle between individual fibres of said structure and water is greater than 90 °.

The present invention relates to a fibrous structure, which may be tested in series of run-off tests, wherein each run-off test comprises exposing said fibrous structure to a volume of a test solution wherein the test solution is test solution 1 which will be described later. Moreover, said fibrous structure is initially hydrophobic and has been treated to be hydrophilic. Furthermore, said fibrous structure exhibits a run-off level that is less than five percent by weight throughout a series of run-off tests wherein the series starts with a first run-off test and ends with a fifth run-off test, and said volume is 25 ml and the test solution after the first run-off test has a surface tension which is less than $60^*10^{-3}$ N/m.

Further, said fibrous structures may consist of one or more non-woven materials. Moreover, the fibrous structures may for instance be used as fluid permeable cover sheet for absorbent articles or as a fluid transfer layer between the fluid permeable cover sheet and an absorbent structure (the absorption body) in an absorbent article, or for the absorbent structure itself. Further, said one or more fibrous structures may constitute a part or all of the fluid pervious cover layer and/or of a fluid transfer layer positioned between the fluid pervious cover layer and the absorption body. Still further, the fibrous structures may comprise one or more tissue layers.

Further the fibrous structure may be used for tissue products. As well known to the man skilled in the art, the term "tissue" commonly covers fibrous material based on cellulose or cellulose in combination with synthetic fibres. Tissue are typically used in the manufacture of household items such as kitchen towels, toilet paper, napkins or wipes, in the manufacture of layers entering the structure of absorbent articles such as diapers, incontinence guards, sanitary napkins, or the like.

However, said fibrous structure is initially hydrophobic and has been treated to be hydrophilic.

Further, a fibrous structure according to the invention is initially hydrophobic and has been treated to be hydrophilic and exhibits a run-off level that is less than two percent by weight throughout the series of run-off tests, as defined herein, wherein the series starts with a first run-off test and ends with a fifth run-off test.

Still further, a fibrous structure according to the invention as described herein exhibits a run-off level that is zero throughout the series of run-off tests, as defined herein, wherein the series starts with a first run-off test and ends with a fifth run-off test.

Furthermore, a fibrous structure article according to the invention as described herein exhibits the afore-mentioned run-off level throughout said series of run-off tests, wherein said fibrous structure has been accomplished by for example bounding one or more types of polar, silicon-containing compounds to at least one portion of the surface of the fibrous structure.

Even further, a fibrous structure article according to the invention as described herein exhibits the afore-mentioned run-off level throughout said series of run-off tests, wherein said fibrous structure has been accomplished by for example bounding one or more types of polar, silicon-containing compounds to at least one portion of the surface of the fibrous structure and adding a surface active agent or a composition comprising a surface active agent. Further, an alternative to accomplish said fibrous structure may be to add a surface active agent or a composition comprising a surface active agent as an internal additive to the fibrous structure prior to the bounding of said silicon-containing compounds.

In general, materials with added surface active agents act hydrophilic or decrease the surface tension of the liquid in contact with said material. Moreover, detergents, wetting agents (surfactants) and emulsifiers are the three general categories of surface active agents.

The surface active agent as used herein may be composed of one surface active agent or may be any suitable mixture of two or more surface active agents. Further, the surface active agent may be, for example, any suitable anionic, cationic, nonionic or zwitterionic surface active agents, i.e. salts of quaternary amines or fatty acids, sulphates, sulphonates, amines or derivatives thereof, polymeric surfactants, or any suitable amphiphilic proteins.

In anionic, cationic, zwitterionic and nonionic surface active agents the hydrophobic part may be a hydrocarbon chain, a polyoxypropylene chain, perfluoroalkyl or polysiloxane; the anionic part may be carboxylic acid salts, sulphonic acid salts, sulphuric acid ester salts, phosphoric or polyphosphoric esters, or perfluorinated anions; the cationic part may be long-chain amines, diamines, polyamines, or any suitable salts thereof, quaternary ammonium salts, polyoxyethylenated (POE) long-chain amines, quaternized polyoxyethylenated (POE) long-chain amines, or amine oxides; the non-ionic part may be polyoxyethylenated alkylphenols, alkylphenol "ethoxylates" (APE), polyoxyethylenated straight chain alcohols, alcohol "ethoxylates" (AE) polyoxyethylenated polyoxypropylene glycols, polyoxyethylenated mercaptans, long chain carboxylic acid esters, alkanolamine "condensates" alkanolamides, polyoxyethylenated silicones, n-alkylpyrrolidones or alkyl polyglycosides; and the zwitterionic part may be pH-sensitive, i.e. β-n-alkylaminopropionic acids, n-alkyl-β-iminodipropionic acids, imidozoline carboxylates, n-alkylbetaines or amine oxides, or pH-insensitive, e.g. sulfobetaines.

A composition comprising a surface active agent may be for example a lotion; a hydrophilic glue, a hydrophilic skin care product; and/or similar.

The hydrophilic glue which is used to achieve the desired properties of said fibrous structures may be e.g. a dispersion glue or a modified hydrophobic glue (i.e. a melting glue) which is modified to be hydrophilic.

Further, when the surface active agent or the composition comprising the surface active agent is added as an internal additive to the fibrous structure prior to the bounding of said silicon-containing compounds, the surface active agent has enough time to migrate out to the surface of the fibrous structure to secure an immediate first inlet. However, it is important in the case of internal additives that the bounding of said silicon-containing compounds to the fibrous structure is perfomed before the internal additive has migrated to the surface, or otherwise a cleaning step is required.

Further, a fibrous structure according to the invention, exhibits excellent properties of "hydrophilicity" or "wettability" which are both immediate and durable.

Further, the invention relates to a fibrous structure having one or more types of polar, silicon-containing compounds bounded to at least one portion of the surface of said fibrous structure, and wherein a surface active agent or a composition comprising a surface active agent is added to the fibrous structure. The surface active agent or the composition comprising the surface active agent may be of any suitable kind as described earlier and is added for example by a conventional spraying technique, a conventional foaming technique, a conventional kiss-roll technique or is added as an internal additive.

The present invention provides a fibrous structure of the kind mentioned in the introduction, wherein said fibrous structure exhibits when desired a well defined rate of wetting, i.e. a predetermined degree of hydrophilicity which is substantially unaffected by wetting of the fibrous structure.

Furthermore, with the present invention a fibrous structure is provided having with a desired, predetermined degree of hydrophilicity which is maintained even after said fibrous structure has been stored for a period of time.

A fibrous structure, in accordance with the invention is primarily distinguished by one or several types of polar, silicon-containing compounds being bound to at least one portion of the surface of said fibrous structure by interaction between the surface and the silicon containing compounds, and by the addition of a surface active agent or a composition comprising a surface active agent to said fibrous structure.

As previously mentioned, the fibrous structure according to the invention exhibits a predetermined degree of hydrophilicity properties which is substantially unaffected by wetting of said fibrous structure.

In accordance with one embodiment, the silicon-containing compound consists of a compound of the type $SiO_xH_y$ wherein x preferably is in the range of 1 to 4, and y preferably is in the range of 0 to 4.

An advantage with a fibrous structure of this type is that the wetting characteristics of the structure has proved to be substantially constant during wetting and that the fibrous structure is comparatively resistant to ageing.

As already mentioned, the fibrous structures in accordance with the invention exhibit at least one polar silicon-containing material surface, or portion of a surface and wherein a surface active agent or a composition comprising a surface active agent is added to the fibrous structure. However, it is possible according to the invention to apply silicon-containing compounds, and wherein a surface active agent or a composition comprising a surface active agent is added to both surfaces of a sheet of material. Further, one or both surfaces of the material may exhibit one or more delimited areas having polar silicon-containing compounds and wherein a surface active agent or a composition comprising a surface active agent is added.

According to one fibrous structure of the invention, said fibrous structure consists of one or more non-woven materials. According to one further aspect of the invention, the fibrous structure may, for instance, be used as fluid permeable cover sheet for absorbent articles or as a fluid transfer layer between the fluid permeable cover sheet and the absorbent structure (absorption body) in an absorbent article, or for the absorbent structure itself.

The invention additionally relates to a fibrous structure for use in an absorbent article such as a diaper, an incontinence guard, a sanitary napkin, wipe or the like, said absorbent article comprises an absorption body being enclosed between a fluid impermeable cover layer and a fluid permeable cover layer (fluid permeable cover sheet), said article comprising at least one portion comprising a fibrous structure as described herein.

Further the fibrous structure may be used for tissue products. As well known to the man skilled in the art, the term "tissue" commonly covers fibrous material based on cellulose or cellulose in combination with synthetic fibres. Tissue are typically used in the manufacture of household items such as kitchen towels, toilet paper, napkins or wipes, in the manufacture of layers entering the structure of absorbent articles such as diapers, incontinence guards, sanitary napkins, or the like.

The present invention also relates to a fibrous structure as described herein, wherein said fibrous structure constitutes a fluid permeable cover sheet, or one or more layers of a multi-layered fluid permeable cover sheet of an absorbent article.

In accordance with one embodiment, the treatment, besides the addition of surface active agent or a composition comprising a surface active agent, is based on a electrical discharge led in a gaseous mixture, leading to the formation of a plasma.

As well known, a plasma is a gaseous medium containing ions, radicals, electrons, excited, metastable and unstable species. It can be obtained through supplying to a gaseous mixture a sufficient amount of energy, at a defined pressure, for example very low pressure or atmospheric pressure.

All the species of the plasma can react between them and/or with the components of the gaseous mixture to create new ions, radicals, and excited species.

When it is carried out at atmospheric pressure with a high voltage electrical energy supply, the plasma is commonly called "corona".

According to this embodiment, the fibrous structure is therefore submitted to a treatment atmosphere as obtained by an electrical discharge applied to a gaseous mixture comprising at least one type of silicon-containing gaseous compound, oxygen or other oxygen-containing gas, and a carrier gas.

In any case, the unstable and excited species of the atmosphere react with the polymer chains of the surface of the fibrous structure, leading to the formation of radicals of said polymer chains. The radicals can then react with species present in the vicinity of the radicals and thereby forming new bondings and new functional groups on the surface. Functional groups which are relevant to the present invention are polar silicon-containing groups. The functional groups which are introduced on the surface by the reaction of the radicals, are much more strongly bound to the surface than an active substance which has been applied as a conventional coating.

A method for corona treatment is described in US 5,576,076, US 5,527,629 and US 5,523,124. The gas mixture is based on a carrier gas which usually is nitrogen, a silicon-containing compound and an oxidant. The treatment creates a layer of material having an inorganic, hydrophilic surface.

The disclosed method is suitable for use in connection with the invention. However, the invention is not limited to the method described in the above mentioned applications, but comprises all types of gas phase treatments in which polar silicon-containing groups are introduced to a surface of a fibrous structure, and wherein a surface active agent or a composition comprising a surface active agent is added to the fibrous structure.

In accordance with one embodiment the silicon-containing compound in the gas mixture is a silane compound.

Some examples of such compounds are $Si_nH_{2n+2}$ where n preferably is from 1 to 4, silicon hydrides, halogenated silanes, alkoxysilanes or organosilanes. The oxidant may be oxygen or other oxygen-containing gases such as, for instance, CO, $CO_2$, NO, $N_2O$ or $NO_2$. The carrier gas may consist of nitrogen, argon, helium, or any mixture thereof.

According to one embodiment of the invention, prior to being treated with the medium comprising unstable and excited species, resulting from the application of an electrical discharge to the gaseous mixture comprising the silicon-containing gaseous compound, an oxidant and a carrier gas, the fibrous structure has been in a first step submitted to a corona discharge under air or under an atmosphere comprising a carrier gas and an oxidizing gas (surface preparation).

According to a further embodiment of the invention said fibrous structure, after being submitted to said electrical discharge in presence of said gaseous mixture, has been submitted to a post-treatment by being submitted to a corona discharge under air or under an atmosphere comprising a carrier gas and an oxidizing gas.

The present invention also relates to use of a fibrous structure as described herein for making tissue products.

Further, the present invention relates to a tissue product made of a fibrous structure as described herein.

Furthermore, the present invention relates to a nonwoven material made of a fibrous structure as described herein.

Other characteristics and advantages of the present invention will become apparent from the following detailed description of some of the embodiments thereof in connection with the appended drawings.

SHORT DESCRIPTION OF FIGURE

The invention will in the following be described in greater detail with reference to the embodiment which is shown in the appended drawing.

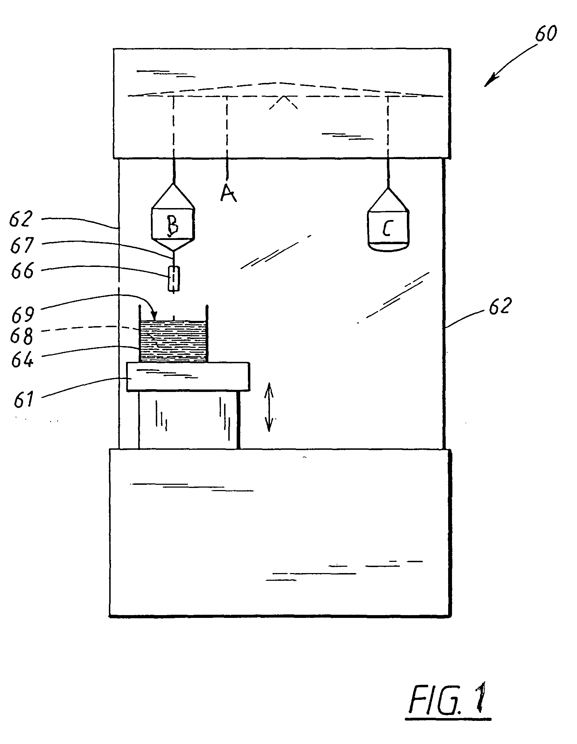

Figure 1 shows means for determining the surface tension (i.e. surface energy) of a test-solution

DESCRIPTION OF EMBODIMENTS AND EXAMPLES

Moreover according to the invention, said bounding of one or more types of polar, silicon-containing compounds to at least one portion of the surface of said fibrous structure increases the surface energy of said fibrous structure, which renders less migration of the added surface active agent or the composition comprising a surface active agent due to a higher affinity/attraction of the added surface active agent or the composition comprising a surface active agent to the more polar surface of said fibrous structure, and as a consequence of said less migration giving fibrous structures and absorbent article less sensitive to ageing.

Furthermore a smaller amount of surface active agent or a composition comprising a surface active agent can be used, and/or a more homogeneously "coated" fibrous structure can be obtained.

Further, as it is possible according to the present invention, to delimit said addition of a surface active agent or a composition comprising a surface active agent, it is also possible to delimit any problems related to migration for example skin irritation, influence of any absorption bodies, or ageing of said absorbent articles.

Accordingly, the present invention provides a means for creating e.g. a fluid permeable cover sheet which, besides exhibiting excellent properties of "hydrophilicity" or "wettability" which are both immediate and durable, does not show any of or at least significantly reducing the problems related to conventional surface active agent treatment or to commonly used corona treatment (optionally in combination with addition of hydrophilic substances) described in the background art, because of said bounding of one or more types of polar, silicon-containing compounds and said addition of a surface active agent or a composition comprising a surface active agent.

Example 1 - Preparation of fibrous structures

In the samples 1A-E below the fibrous structures comprise a non-woven polypropylene with a basis weight of 18 g/m2.

1A. polypropylene non-woven material which has been treated with surface active agent

1B. polypropylene non-woven material which has been corona treated with a conventional method and surface active agent is added to the material

1C. polypropylene non-woven material which has been corona treated in order to introduce polar silicon-containing groups to the surface of the material and surface active agent is added to the material

1D.   polypropylene non-woven material and 0.23 weight % of surface active agent is added to the material

1E.   polypropylene non-woven material which has been corona treated in order to introduce polar silicon-containing groups to the surface of the material

1F.   polypropylene non-woven material which has been corona treated in order to introduce polar silicon-containing groups to the surface of the material and 0.14 weight % of surface active agent is added to the material

Sample 1 B was here treated in one step: corona treated under air.

The operating conditions under which the sample 1B was treated according to the invention are as follows:

Speed of the web (the fibrous structure) = 22 m/min
Width of the electrode = 0.65 m
Electrical power of corona = 1720 W

The operating conditions under which the samples 1C, 1E and 1F were treated according to the invention are as follows:

Speed of the web (the fibrous structure) = 25 m/min
Width of the electrode = 0.65 m
Electrical power of corona = 1463 W
Flow rate of $N_2$ = 166 l/min
Flow rate of $N_2O$ = 0.38 l/min
Flow rate of $SiH_4$ = 0.110 l/min

Samples 1C, 1E and 1F were treated in two steps: in a first step, corona treated under air, and in a second step, corona treated with injection of the above described gaseous mixture of $N_2$, $N_2O$ and $SiH_4$.

Further, to samples 1A, 1B and 1C a surface active agent (an organosilicon based surfactant with trade name Nuwet 237) was added by spraying a 0.5 weight % water solution of the surface active agent to the fibrous structures by a conventional spraying technique, to give samples having $1.0 \pm 0.2$ weight % of the surface active agent calculated on dry weight of said samples. The amount of surface active agent added to samples 1A, 1B and 1C was measured by an "Extraction method".

Said "Extraction method" comprises the following steps: Weighing 8.0 g of the sample, and adding said 8.0 g of the sample and 200 ml isopropanol to a pre-weighed beaker. Stirring every fifteen minute and letting the sample and the isopropanol be in the beaker for one hour at room-temperature. Wringing out the isopropanol from the sample and moving the sample from the beaker. Letting the isopropanol in the beaker evaporate and weighing the beaker giving the amount of surface active agent. Further, letting the sample dry and weighing the sample giving the dry weight of the sample.

Moreover, sample 1D is a commercially available material which has 0.23 weight % of a surface active agent (an organosilicon based surfactant with trade name Nuwet 237) added, to sample 1E there was no surface active agent added, and to sample 1F 0.14 weight % of a surface active agent (an organosilicon based surfactant with trade name Nuwet 237) was added by using a kiss-roll technique. The amount of surface active agent added to samples 1D and 1F was measured by a "Wet Pick-Up"-method and is given as weight % of dry weight of said samples. The "Wet Pick-Up"-method involves weighing the dry and the wet sample. The wet sample is weighed directly after application of the surfactant solution to the sample. Knowing the concentration of the surfactant solution, the weight of the applied surfactant can be calculated.

After the addition of surface active agent, samples 1A, 1B, 1C, 1D and 1F were dried.

Example 2 - Determination of run-off level in percent by weight run-off for a fibrous structure

Percent by weight run-off of samples 1A, 1B and 1C were determined in a first round, and percent by weight run-off of samples 1D, 1E and 1F were determined in a second round.

In order to determine percent by weight run-off for a fibrous structure a run-off test, the EDANA test method no. 152.00-99, was used. By the run-off test the amount of non-absorbed fluid (test solution 1) is measured when a determined amount, volume is 25 ml, of the test solution 1 is poured onto the fibrous structure from a height of 25 mm and at a flow rate of 7 ml/s.

Samples of the fibrous structures, non-woven polypropylene with a basis weight of 18 $g/m^2$, 140x285 mm is prepared by cutting and the long side is in the machine direction. The fibrous structure is fixed and placed on top of three layers of filter paper (FFS filter paper 140 x 280 mm from Hollingsworth & Vose Company Ltd) on an inclined table and the inclination is 25°. The length of each tested fibrous structure from the point where the test solution 1 is inserted to the end of the fibrous structure in the run-off direction is 260 mm.

The amount of the test solution 1 which is not absorbed by the fibrous structure, i.e. the run-off, is collected and weighed. The run-off test is repeated five times on the same fibrous structure with a four minutes interval. The run-off is weighed after each run-off test. Thus, the run-off is measured five times after each other on the same fibrous structure in a series of run-off tests wherein the series starts with a first run-off test and ends with a fifth run-off test. The three layers of filter paper is replaced after each run-off test in the series.

The test solution 1 used in the test method is a 0.9 weight percent (NaCl) saline solution having a surface tension above $70^{*}10^{-3}$ N/m before the first run-off test.

Result of example 2 - run-off level

First round

| Sample | Run-off (% weight) | | | | |
|---|---|---|---|---|---|
| | 1st test | 2nd test | 3rd test | 4th test | 5th test |
| 1A | 0 | 1 | 16 | 56 | 74 |
| 1B | 0 | 0 | 4 | 18 | 55 |
| 1C | 0 | 0 | 0 | 0 | 0 |

Second round

| Sample | Run-off (% weight) | | | | |
|---|---|---|---|---|---|
| | 1st test | 2nd test | 3rd test | 4th test | 5th test |
| 1D | 0 | 4 | 41 | 72 | 64 |
| 1E | 71 | 60 | 33 | 31 | 23 |
| 1F | 0 | 0 | 0 | 0 | 0 |

Example 3 - Determination of surface tension of test solution 1

The surface tension (i.e. surface energy) of the test-solution was determined by means of the apparatus shown in figure 2. Thereby, Wilhelmy's method was used.

The balance that was used for the determination of the surface tension in connection with the invention, is manufactured by Cahn instruments in California. The model number is DCA-322, where DCA stands for "Dynamic Contact Angle". A PC, IBM300PL, was used for controlling the instrument. The same computer was also utilized for recording data from the measurements and for performing the subsequent calculations.

Before measuring a lamina of glass 66 is properly cleaned by burning in a flame. During the measurement, the lamina of glass 66 is vertically suspended in an extremely sensitive balance 60. A liquid container 64 is placed on a mobile table 61, directly below the lamina of glass 66. When the lamina of glass 66 is dipped into the liquid 68, a liquid meniscus, which affects the partially immersed lamina with a vertical force, is formed around the lamina.

The force is measured by means of the balance . The force is related to the surface tension according to:

$$F = \gamma p \cos\theta + mg - \rho_L \, lgA$$

F= the measured force (N)
$\gamma$=the surface tension (i.e. surface energy) of the liquid (N/m)
p= the circumference of the fibre (m)
$\theta$= the contact angle in the interface solid-lamina of glass
m= the mass of the solid (kg)

g= the constant of gravitation (m/s2)
$\rho_L$ = the density of the liquid (kg/m3)
I= the depth of immersion of the solid (m)
A=the cross-sectional area of the solid (m3)

The second term in the equation represents the weight of the mounted fibre, while the third term of the equation is the so-called "buoyancy-force", i.e. the weight loss which arises as a result of displaced liquid volume. In a computer (not shown) furnished with a calculation program for surface tension determination, usually both these two terms are taken into account, something which simplifies the equation to:

$$F=\gamma p\cos\theta$$

The surface tension of the liquid is obtained by measuring the force when the lamina is lifted up from the liquid.
The mobile table 61 is elevated and lowered with a constant speed (150 μm/s). Furthermore, the temperature in the sample chamber should be controlled.
The balance 60 has three pans (see fig. 2). The pan B with an accuracy of $10^{-5}$ g, is used for surface tension (surface energy) measurements on liquids. The balance is tared by means of placing counterweights in a third pan C.
In order to prevent draughts, dust, or the like, from disturbing the measurement, the pans and the mobile table 61 are protected by sliding glass frames 62. These also enable control of air moisture and temperature. In order to avoid disturbing vibrations during the course of the measurement, the balance is placed on a foundation (not shown).
The table which the liquid container 64 stands on is elevated and lowered by means of a motor (not shown). The speed of the table 61 is controlled by the connected computer and is displayed before a measurement is started. Other parameters which have to be fed in before the measurement is started are the maximal depth and speed of immersion. The software of the computer use a contact angle of zero for surface tension (surface energy) calculation of liquids.
The balance 60 has first been tared with the metal clamp 67 and the lamina being suspended in the pan B. Test liquid 68, is placed in the liquuid container 64 on the table 61 below the lamina. The lamina should be suspended perpendicular to the liquid surface 69 and has to be completely still before the measurement starts so that the balance shows a stable value. The table 61 with the liquid container 64 is elevated so that the liquid surface 69 is approximately 1 mm from the lamina.
When the measurement is started, the computer records a base line, whereafter the table 61 is elevated with a predetermined speed. When the maximal depth of immersion is reached, the computer is commanded to lower the table.
The surface tension was determined for test solution 1 after a first run-off test material on sample 1D, 1E and 1F respectively. Said first run-off test was achieved as in Example 2 above with the exception that there was no filter paper used, thus enabling collection of test solution after first run-off test.

1D.    polypropylene non-woven material and 0.23 weight % of surface active agent is added to the material

1E.    polypropylene non-woven material which has been corona treated in order to introduce polar silicon-containing groups to the surface of the material

1F.    polypropylene non-woven material which has been corona treated in order to introduce polar silicon-containing groups to the surface of the material and 0.14 weight % of surface active agent is added to the material

Results of example 3 - surface tension (i.e. surface energy) of the liquid (N/m)

| Sample | surface tension (N/m) |
|--------|----------------------|
| 1D | $51^*10^{-3}$ |
| 1E | $71^*10^{-3}$ |
| 1F | $53^*10^{-3}$ |

Before said first run-off test, the surface tension of test solution 1 is above $70^*10^{-3}$ N/m.
The invention shall not be regarded as being restricted to the embodiments which have been described herein.

Accordingly, a plurality of further variants and modifications are conceivable within the scope of the appended claims.
Therefore, if the invention and all its advantages have been particularly described and illustrated in the case of non-woven fibrous structures, and in the case of absorbent articles, as will be clearly apparent to the man skilled in the art, the invention finds a much larger field of application, including absorbent articles comprising for example woven fibrous structures, of either the natural or synthetic type.

**Claims**

1. A fibrous structure, being testable in a series of run-off tests, wherein each run-off test comprises exposing said fibrous structure to a volume of a test solution and said fibrous structure initially is hydrophobic and has been treated to be hydrophilic, **characterized in that** said fibrous structure exhibits a run-off level that is less than five percent by weight throughout a series of run-off tests wherein the series starts with a first run-off test and ends with a fifth run-off test, and said volume is 25 ml and said test solution after the first run-off test has a surface tension which is less than $60*10^{-3}$ N/m.

2. A fibrous structure according to claim 1, **characterized in that** said fibrous structure exhibits a run-off level that is zero throughout the series of run-off tests.

3. A fibrous structure according to claim 1 or 2, **characterized in that** said fibrous structure has been accomplished by bounding one or more types of polar, silicon-containing compounds to at least one portion of the surface of the fibrous structure.

4. A fibrous structure according to any of claims 1 to 3, **characterized in that** said fibrous structure has been accomplished by bounding one or more types of polar, silicon-containing compounds to at least one portion of the surface of the fibrous structure and adding a surface active agent or a composition comprising a surface active agent.

5. A fibrous structure having one or more types of polar, silicon-containing compounds bounded to at least one portion of the surface of said fibrous structure, **characterized in that** a surface active agent or a composition comprising a surface active agent is added to said fibrous structure.

6. A fibrous structure according to any of the preceding claims, **characterized in that** said silicon-containing compounds are of the type $SiO_xH_y$ wherein x preferably is in the range of 1 to 4, and y preferable is in the range of 0 to 4.

7. A fibrous structure according to any of the preceding claims, **characterized in that** said fibrous structure has been submitted to an electrical discharge, in presence of a gaseous mixture comprising at least one type of silicon-containing gaseous compound, oxygen or other oxygen-containing gas, and a carrier gas.

8. A fibrous structure according to claim 7, **characterized in that** said fibrous structure, prior to being submitted to said electrical discharge, in presence of said gaseous mixture, has been in a first step submitted to a corona discharge under air or under an atmosphere comprising a carrier gas and an oxidizing gas.

9. A fibrous structure according to claim 7 or claim 8 **characterized in that** said fibrous structure, after being submitted to said electrical discharge in presence of said gaseous mixture, has been submitted to a post-treatment by being submitted to a corona discharge under air or under an atmosphere comprising a carrier gas and an oxidizing gas.

10. Use of a fibrous structure according to any of the preceding claims for making tissue products.

11. A tissue product made of a fibrous structure according to any of claims 1 to 9.

12. A nonwoven material made of a fibrous structure according to any of claims 1 to 9.

FIG. 1

# EUROPEAN SEARCH REPORT

**European Patent Office**

**Application Number**

EP 00 85 0022

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | EP 0 483 859 A (KIMBERLY CLARK CO) 6 May 1992 (1992-05-06) * the whole document * | 1,2, 10-12 | D06M10/02 D06M15/643 D06M10/10 |
| Y | | 1-4 | |
| X | EP 0 372 890 A (CHISSO CORP) 13 June 1990 (1990-06-13) * column 1, line 39 - line 46 * | 5,10-12 | |
| Y | * column 4, line 25 - column 5, line 32 * | 1-4 | |
| A | DATABASE WPI Section Ch, Week 198431 Derwent Publications Ltd., London, GB; Class A35, AN 1984-191108 XP002142851 & JP 59 106570 A (EMORI SHOJI KK), 20 June 1984 (1984-06-20) * abstract * | 5,7 | |
| A | WO 99 05358 A (SAYERS IAN CHRISTISON ;SCAPA GROUP PLC (GB)) 4 February 1999 (1999-02-04) * the whole document * | 7 | TECHNICAL FIELDS SEARCHED (Int.Cl.7) D06M |

The present search report has been drawn up for all claims

| Place of search | Date of completion of the search | Examiner |
|---|---|---|
| THE HAGUE | 18 July 2000 | Bernardo Noriega, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**   EP 00 85 0022

This annex lists the patent family members relating to the patent documents cited in the above-mentioned European search report.
The members are as contained in the European Patent Office EDP file on
The European Patent Office is in no way liable for these particulars which are merely given for the purpose of information.

18-07-2000

| Patent document cited in search report | Publication date | Patent family member(s) | Publication date |
|---|---|---|---|
| EP 0483859 A | 06-05-1992 | US 5112690 A | 12-05-1992 |
| | | AU 646303 B | 17-02-1994 |
| | | AU 8693391 A | 07-05-1992 |
| | | CA 2054086 A | 02-05-1992 |
| | | DE 69111561 D | 31-08-1995 |
| | | DE 69111561 T | 18-01-1996 |
| | | ES 2077765 T | 01-12-1995 |
| | | KR 188056 B | 01-06-1999 |
| | | MX 9101726 A | 08-07-1992 |
| | | ZA 9108215 A | 26-08-1992 |
| EP 0372890 A | 13-06-1990 | JP 2169774 A | 29-06-1990 |
| | | JP 2613798 B | 28-05-1997 |
| | | DE 68913280 D | 31-03-1994 |
| | | DE 68913280 T | 26-05-1994 |
| | | DK 616289 A | 09-06-1990 |
| | | US 5087520 A | 11-02-1992 |
| JP 59106570 A | 20-06-1984 | JP 1044835 B | 29-09-1989 |
| | | JP 1560029 C | 31-05-1990 |
| WO 9905358 A | 04-02-1999 | AU 8452698 A | 16-02-1999 |
| | | EP 0998606 A | 10-05-2000 |